# EUROPEAN PATENT SPECIFICATION

(11) **EP 2 776 072 B1**
(45) Date of publication and mention of the grant of the patent: **03.06.2015**
(21) Application number: 12716634.6
(22) Date of filing: 29.03.2012
(51) Int. Cl.: A61L 2/02, B24C 1/00, B24C 5/04, A61B 19/00

(54) **PROCESS FOR CLEANING AND SANITIZING SURGICAL INSTRUMENTS IN GENERAL AND DEVICE SUITED TO IMPLEMENT SAID PROCESS**
VERFAHREN ZUM REINIGEN UND DESINFIZIEREN VON CHIRURGISCHEN INSTRUMENTEN IM ALLGEMEINEN UND VORRICHTUNG ZUR DURCHFÜHRUNG DIESES VERFAHRENS
PROCÉDÉ DE NETTOYAGE ET D'ASSAINISSEMENT D'INSTRUMENTS CHIRURGICAUX D'UNE MANIÈRE GÉNÉRALE ET DISPOSITIF APPROPRIÉ POUR METTRE EN OEUVRE LEDIT PROCÉDÉ

(30) Priority: 11.11.2011 IT PD20110352
(43) Date of publication of application: 17.09.2014
(73) Proprietor: Bicar Jet S.r.l., 35129 Padova (IT)
(72) Inventor: SOMMACAL, Alessandro Paolo, I-35129 Padova (IT)
(74) Representative: Vinci, Marcello
(86) International application number: PCT/IB2012/051514
(87) International publication number: WO 2013/068857

(56) References cited:
- EP-A1- 1 683 499
- WO-A2-02/092283
- US-A- 5 177 911
- US-A- 5 556 324

## Description

The present patent concerns the processes and devices for cleaning surgical tools or surgical instruments, and in particular it concerns a new sand blasting process for cleaning and sanitizing surgical tools or surgical instruments in general, and a device suited to implement said process.

Surgical tools and instruments used in the medical field, for example in surgery, dentistry, veterinary medicine, etc, are known, which are used for carrying out operations on the patient's body.

In order to be used, said instruments must be completely and accurately cleaned and sanitized, meaning that their surface must not present any type of foreign substance, bacteria or other elements that may cause pathological reactions in any type of patient.

Therefore, surgical tools must be cleaned again and sanitized accurately after each use, as well as packaged individually in sterile and sealed packages suited to be quickly opened to extract the tools at the moment of use by the surgeon or operator in general.

In particular, before undergoing the sanitization process, which usually is performed with high temperature steam or other chemical or thermal systems, the surgical instruments must be previously cleaned very accurately in order to completely remove any organic residues from their metal surface.

It is in the organic residues, in fact, if they are not removed before sanitization, that the molecule breaking process takes place during the heat treatment, with the formation of substances that affect the cleanliness and sterility of the surgical instrument and considerably increase corrosion problems.

In order to protect the health of medical personnel against possible infections due to contact, even if accidental, with infected surgical instruments, automatic instrument washing devices have been designed that are used for cleaning and sanitizing the instruments.

These devices usually operate in three steps. During the first step the surgical instruments are washed with suitable detergents and rinsed with water, which must ensure complete removal of the organic residues present on the instruments.

A drawback posed by the known processes using chemical products lies in that some of them, commonly used in washing cycles, are too aggressive against the stainless steel used for making surgical instruments and cause these to corrode.

Furthermore, the surgical instruments comprise portions of their surface that are smooth and other portions that are rough, for example at the level of the handpieces, where better hold must be guaranteed to the operator. Some surgical instruments also comprise interstices and small spaces according to the specific shape of the instrument and to usage needs.

Organic impurities accumulate in said interstices and on said rough surface and are difficult to remove with the known washing processes.

Due to the fact that the cleansing systems used are not perfectly effective, before the washing operations a manual mechanical operation is often necessary and this must be performed by a specialized operator. This operation consists in the manual brushing of the instruments, which subjects the operator to the risk of infections, due to possible cuts, abrasions, punctures or any other accidental event deriving from the brushing operation. Furthermore, the brushing operation does not always guarantee the complete removal of organic residues, since the tools used for this mechanical operation cannot always reach the contaminated interstices or cavities and/or sometimes the encrustation resulting from the drying of the organic substances are specially adherent to the surface.

For this reason systems are also known that use softening baths suited to favour the mechanical and/or chemical removal of organic residues from the most complex surfaces, however these methods are not always effective. The contaminations left, especially in the interstices and cavities, are then fixed with the sanitizing heat treatment and therefore the effectiveness of this last step, so important for the health of the patients that will be treated subsequently, cannot be guaranteed.

The second, neutralizing step, includes the use of acid products that serve to solubilize and completely remove the oxides formed during cleaning with detergents.

The acid solutions used, however, have also a corrosive effect, in particular at the level of the contact points between metal and metal or between metal and plastic, where the conditions may facilitate the occurrence of localized corrosion phenomena.

The third step includes the cleansing of the instruments with high temperature water, approximately at 90°, which may cause more marked corrosion effects at the level of said areas subjected to localized corrosion.

EP1683499 relates to a method for treating medical instruments and to the corresponding device using sand as abrasive material. The use of sand as abrasive material involve a corrosive effect on the metallic surface of the instruments. Furthermore, sand is not biodegradable and it is not soluble and therefore it is polluting and thus subjected to special restrictions after used.

Therefore, the known processes and equipment for cleaning and sanitizing surgical instruments pose several drawbacks.

In order to overcome said drawbacks, a new type of sand blasting process has been studied and implemented, which uses sodium bicarbonate for cleaning and sanitizing surgical tools or surgical instruments in general, in addition to a device for implementing said process.

The main object of the present invention is to ensure highly effective mechanical removal of the substances stuck to the entire surface of the instruments and, at the same time, a highly effective washing, sanitizing and disinfecting process, if any.

It is a further object of the present invention to guarantee a long-lasting disinfecting and anti-bacterial action, intended to hinder a new proliferation of bacteria on the surfaces of the instruments.

A further important advantage of the present invention also consists in the considerable reduction of washing times and thus of the related costs, as the new product performs the abrading step, the cleansing step and if necessary also the disinfecting and/or biocidal action at the same time, with a single application.

Another advantage deriving from the use of the present invention lies in the reduction of energy costs, as the effectiveness of the product is guaranteed even if the product is applied with air, with or without water, not heated, and thus with no need to use hot water.

These and other direct and complementary objects are achieved by the new sand blasting process which uses sodium bicarbonate for cleaning and sanitizing surgical tools or surgical instruments in general, and by a device for implementing said process.

According to the new cleaning process, the removal of the substances adhering and stuck to the surfaces of the instrument to be cleaned takes place through abrasion, with the ejection of abrasive cleaning material at high speed against the instruments to be cleaned. The invention is defined by claims 1 and 8.

Said abrasive cleaning material comprises sodium bicarbonate salts, mixtures of the same.

Said sodium bicarbonate salts can be used both dry and wet, in the latter case dissolved in water beyond the saturation limit, so as to form a solution comprising dissolved and undissolved salts, suited to be emitted under pressure and at high speed and ejected against the instruments to be cleaned. The portion of sodium bicarbonate salts dissolved in the solution performs the real washing function, dissolving and leaching the organic material present on the surface of the instruments.

The portion of undissolved sodium bicarbonate salts, instead, serves as abrasive material.

The new process is particularly suitable for washing said surgical instruments, as it does not involve any corrosive effect on the material from which said instruments are made and there is no abrasive action, meaning no surface modification of the instrument.

Furthermore, sodium bicarbonate has a strong sanitizing, antibacterial and antifungal action.

Furthermore, sodium bicarbonate is not dangerous for operators and is not detrimental to the environment, is 100% biodegradable and soluble, which means that it is not polluting and not subjected to special restrictions for storage, transport and handling.

According to a possible embodiment of the invention, in the new process said abrasive cleaning material comprises, in addition to sodium bicarbonate salts and water, even one or more further disinfecting and/or biocidal agents, for example in a concentration included between 0.1 and 25%.

In this way, with a single step it is possible to carry out both the instrument washing operation and the disinfecting operation.

According to the invention, said abrasive cleaning material may also comprise one or more further substances with cleansing and/or disinfecting properties and/or one or more specifically acting medical products.

The water used may be heated or at room temperature, since the use of sodium bicarbonate, in particular with the addition of disinfecting and/or biocidal agents, guarantees optimal cleaning and sanitizing results. Therefore, the new process also ensures savings in energy and running costs.

The elimination of the brushing process results in the elimination of the risks for operators deriving from possible infections due to accidental punctures, abrasions or cuts that may occur during the brushing operation, with a clear reduction of any accident related costs.

The improved effectiveness of the cleaning process also generates a considerable reduction in the percentage of infections in patients treated subsequently, thanks to better and more effective cleaning of the surgical instruments, with a consequent reduction of the related costs.

The process may comprise a final step in which the instruments are rinsed with cold water, meaning non heated water, and/or are blown with dry sodium bicarbonate salts.

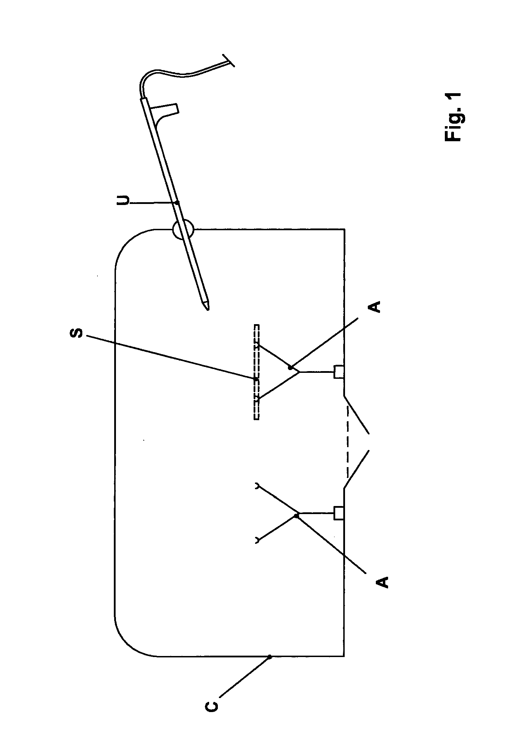

For the implementation of the new process for cleaning and sanitizing surgical instruments a device, shown in Figure 1, is used that comprises a closed sand blasting cabinet (C) suited to accommodate one or more instruments to be cleaned.

According to the invention, inside said cabinet there is/are one or more supports (A) for said surgical instruments (S), suited to constrain the instruments and keep them in position during the sand blasting operation.

Said supports (A) can be of the fixed or movable type, for example they can be rotated or translated, so as to expose the entire surface of the instrument to the jet emitted by the emitter nozzle (U).

According to a possible embodiment of the invention, said emitter nozzle is movable, that is, travels along the three spatial directions and/or rotates, so as to direct the jet on the entire surface of the instruments to be cleaned. Said instruments to be cleaned can in turn be constrained to said fixed or movable supports, or held manually by the user.

Said emitter nozzle can be moved manually or can be automated.

According to a further alternative embodiment, said emitter nozzle is fixed inside the cabinet, and thus the jet is permanently oriented in the same direction. According to this solution, the instruments to be cleaned can be manoeuvred manually by the operator, in order to expose their entire surface to the jet. Alternatively, said supports of the instruments to be cleaned are mechanized and movable, translating and/or rotating, thus completely automating the process.

The device also comprises at least one abrasive material feed system, in turn comprising an abrasive material tank, and wherein, through at least one duct, a flow of pressurized air and/or water draws or thrusts said abrasive material from said tank and transports it to an emitter nozzle located inside said cabinet.

By means of said emitter nozzle said abrasive material is ejected at high speed, inside said sand blasting cabinet, on said instruments to be cleaned.

The device may also comprise a vacuum suction system for drawing the abrasive material inside said cabinet and an air filtering system for filtering the air flowing out of the cabinet and recovering the material used in at least one apposite container.

Said sand blasting cabinet can have any shape and size and comprises a casing with at least one access door for introducing and extracting the surgical instruments.

Said casing of the cabinet comprises also one or more check windows, one or two openings with sleeve glove suited to allow the operator to manoeuvre the sand blasting nozzle and/or to rotate the instruments to be cleaned inside the cabinet, and one discharge opening on the bottom of the cabinet.

Said bottom of the cabinet can be shaped, for example, so as to favour the outflow of the waste material.

The cleaning operations with sodium bicarbonate salts can thus be carried out inside said cabinet, both manually, by one or more operators at the same time, and in an automated manner, with the aid of appropriate instruments or suitable automated mechanical systems.

Therefore, with reference to the above description, the following claims are expressed.

## Claims

1. Process for cleaning and sanitizing surgical tools or surgical instruments in general, comprising the use of abrasive cleaning material, emitted under pressure and at high speed against said instruments for abrading and removing the substances that adhere to the surfaces of the same instruments, **characterized in that** said abrasive cleaning material comprises sodium bicarbonate salts, mixtures of the same.

2. Process (1) according to claim 1, **characterized in that** said cleaning material is used in the dry state.

3. Process according to claim 1, **characterized in that** said abrasive cleaning material comprises said sodium bicarbonate salts dissolved in water beyond the saturation limit so as to form a solution comprising dissolved and/or undissolved salts, suited to be emitted under pressure and at high speed and projected against the instruments to be cleaned, and wherein said water is or is not heated.

4. Process according to claims 1, 2 or 3, **characterized in that** said abrasive cleaning material also comprises one or more further agents with disinfecting and/or biocidal properties.

5. Process according to claim 4, **characterized in that** said disinfecting and/or biocidal agent is present in a concentration included between 0.1 and 25 %.

6. Process according to the preceding claims, **characterized in that** said abrasive cleaning material also comprises one or more further substances with cleansing and/or disinfecting properties and/or one or more specifically acting medical products.

7. Process according to the preceding claims, **characterized in that** it comprises at least one final step for rinsing the instruments with water and/or a blowing step for dry use.

8. Device for cleaning and sanitizing surgical instruments using the process according to the preceding claims, comprising at least one closed sand blasting cabinet (C), said abrasive material comprises sodium bicarbonate salts, mixtures of the same, and said device also comprises:
• said at least one closed sand blasting cabinet (C), suited to accommodate one or more of said instruments (S) to be cleaned, in turn comprising a casing with at least one access door for introducing the instruments (S) to be cleaned;
• at least one system for supplying sodium bicarbonate salts, mixtures of the same, comprising a tank containing sodium bicarbonate salts, mixtures of the same, a duct through which the pressurized air and/or water flow draws or pushes the abrasive material out of said tank to transport it to an emitter nozzle (U) positioned inside said cabinet (C), wherein said emitter nozzle (U) ejects said abrasive material at high speed inside said sand blasting cabinet (C), onto said instruments (S) to be cleaned.

9. Device for cleaning and sanitizing surgical instruments according to claim 8, **characterized in that** said cabinet (C) comprises at least one opening with a sleeve glove suited to allow the operator to manoeuvre said emitter nozzle (U) and/or the instruments (S) to be cleaned inside the cabinet.

10. Device for cleaning and sanitizing surgical instruments according to claims 8, 9, **characterized in that** said emitter nozzle (U) can be moved in an automated way, and translated along the three spatial directions and/or rotated, so as to direct the jet on the entire surface of the instruments (S) to be cleaned.

11. Device for cleaning and sanitizing surgical instruments according to claims 8, 9, **characterized in that** said emitter nozzle (U) is fixed, maintaining the jet direction unchanged.

12. Device for cleaning and sanitizing surgical instruments according to claims 8, 9, 10 or 11, **characterized in that** it comprises one or more supports (A) for said surgical instruments (S) inside said cabinet (C), suited to constrain the instruments (S) and keep them in the correct position during the sand blasting operation.

13. Device for cleaning and sanitizing surgical instruments according to claim 12, **characterized in that** said supports (A) for the instruments (S) to be cleaned can be moved in an automated way, through rotation and/or translation, so as to expose the entire surface of the instrument (S) to the jet of the emitter nozzle (U).

14. Device for cleaning and sanitizing surgical instruments according to claims 8, 9, 10, 11, 12, 13, **characterized in that** it comprises: means for rinsing and/or blowing on the instrument after the sand blasting operation, a vacuum suction system or a pressure thrust system suited to convey the abrasive material into said cabinet (C) and a filtering system for filtering the air that flows out of the cabinet (C) and recovering the used material for which at least one suitable container is provided.

## Patentansprüche

1. Verfahren zur Reinigung und Sanitisierung chirurgischer Werkzeuge oder chirurgischer Instrumente im Allgemeinen, den Gebrauch abrasiven Reinigungsmaterials umfassend, das unter Druck und mit hoher Geschwindigkeit gegen die besagten Instrumente abgegeben wird, um die an den Oberflächen der Instrumente selbst anhaftenden Substanzen abzureiben und zu entfernen, **dadurch gekennzeichnet, dass** das besagte, abrasive Reinigungsmaterial Natriumhydrogencarbonatsalze und Mischungen derselben umfasst.

2. Verfahren gemäß Patentanspruch 1, **dadurch gekennzeichnet, dass** das besagte Reinigungsmaterial im trockenen Zustand verwendet wird.

3. Verfahren gemäß Patentanspruch 1, **dadurch gekennzeichnet, dass** das besagte abrasive Reinigungsmaterial die besagten Natriumhydrogencarbonatsalze über die Sättigungsgrenze hinaus in Wasser gelöst umfassen, so dass eine Lösung entsteht, die gelöste und/oder ungelöste Salze enthält und geeignet ist, unter Druck und mit hoher Geschwindigkeit ausgegeben und gegen die zu reinigenden Instrumente gerichtet zu werden, und wobei das besagte Wasser erhitzt oder nicht erhitzt ist.

4. Verfahren gemäß Patentanspruch 1, 2 oder 3, **dadurch gekennzeichnet, dass** das besagte abrasive Reinigungsmaterial außerdem einen oder mehrere Wirkstoffe mit desinfizierenden und/oder bioziden Eigenschaften enthält.

5. Verfahren gemäß Patentanspruch 4, **dadurch gekennzeichnet, dass** der besagte, desinfizierende und/oder biozide Wirkstoff in einer Konzentration zwischen 0,1 und 25 % enthalten ist.

6. Verfahren gemäß den vorstehenden Patentansprüchen, **dadurch gekennzeichnet, dass** das besagte abrasive Reinigungsmaterial außerdem eine oder mehrere Substanzen mit reinigenden und/oder desinfizierenden Eigenschaften und mit einem oder mehreren, spezifisch wirkenden medizinischen Produkten umfasst.

7. Prozess gemäß vorstehenden Patentansprüchen, **dadurch gekennzeichnet, dass** er wenigstens einen abschließenden Schritt des Abspülens mit Wasser und/oder einen Schritt des Abblasens für den trockenen Gebrauch umfasst.

8. Vorrichtung zur Reinigung und Sanitisierung chirurgischer Instrumente mittels des Verfahrens gemäß vorstehenden Patentansprüchen, wenigstens eine geschlossene Sandstrahlkabine (C) umfassend, wobei das besagte, abrasive Material Natriumhydrogencarbonatsalze und Mischungen desselben umfasst, und die besagte Vorrichtung des Weiteren Folgendes umfasst:
• die besagte, wenigstens eine geschlossene Sandstrahlkabine (C), die geeignet ist, eines oder mehrere der besagten, zu reinigenden Instrumente (S) aufzunehmen und ihrerseits ein Gehäuse mit wenigstens einer Zugangstür zur Einladung der zu reinigenden Instrumente (S) umfasst;
• wenigstens ein System zur Zuführung der Natriumhydrogencarbonatsalze und Mischungen derselben, einen Behälter mit den Natriumhydrogencarbonatsalzen und Mischungen derselben umfassend, sowie eine Leitung, durch welche die Druckluft und/oder ein Wasserfluss das abrasive Material aus dem besagten Behälter zieht oder drückt, um es zu einer Ausgabedüse (U) innerhalb der besagten Kabine (C) zu befördern, wobei die besagte Ausgabedüse (U) das besagte, abrasive Material mit hoher Geschwindigkeit in die besagte Sandstrahlkabine (C) und auf die besagten, zu reinigenden Instrumente (S) ausstößt.

9. Vorrichtung zur Reinigung und Sanitisierung chirurgischer Instrumente gemäß Patentanspruch 8, **dadurch gekennzeichnet, dass** die besagte Kabine (C) wenigstens eine Öffnung mit einem Stulpenhandschuh umfasst, der es dem Bediener erlaubt, die besagte Ausgabedüse (U) und/oder die zu reinigenden Instrumente (S) innerhalb der Kabine zu bewegen.

10. Vorrichtung zur Reinigung und Sanitisierung chirurgischer Instrumente gemäß Patentansprüchen 8 und 9, **dadurch gekennzeichnet, dass** die besagte Ausgabedüse (U) automatisch bewegt und entlang der drei räumlichen Richtungen verschoben und/oder gedreht werden kann, um den Strahl auf die gesamte Oberfläche der zu reinigenden Instrumente (S) zu richten.

11. Vorrichtung zur Reinigung und Sanitisierung chirurgischer Instrumente gemäß Patentansprüchen 8 und 9, **dadurch gekennzeichnet, dass** die besagte Ausgabedüse (U) feststehend ist und die Strahlrichtung unverändert bleibt.

12. Vorrichtung zur Reinigung und Sanitisierung chirurgischer Instrumente gemäß Patentansprüchen 8, 9, 10 oder 11, **dadurch gekennzeichnet, dass** sie eine oder mehrere Halterungen (A) für die besagten, chirurgischen Instrumente (S) in der besagten Kabine (C) umfasst, die geeignet sind, die Instrumente (S) während des Sandstrahlvorgangs zu blockieren und korrekt in Position zu halten.

13. Vorrichtung zur Reinigung und Sanitisierung chirurgischer Instrumente gemäß Patentanspruch 12, **dadurch gekennzeichnet, dass** die besagten Halterungen (A) für die besagten, zu reinigenden Instrumente automatisch durch Drehung und/oder Verschiebung bewegt werden können, um die gesamte Oberfläche der Instruments (S) dem Strahl der Ausgabedüse (U) auszusetzen.

14. Vorrichtung zur Reinigung und Sanitisierung chirurgischer Instrumente gemäß Patentansprüchen 8, 9, 10, 11, 12, 13, **dadurch gekennzeichnet, dass** sie Folgendes umfasst: Mittel zum Abspülen und/oder Abblasen des Instruments nach dem Sandstrahlvorgang, ein Unterdruck-Ansaugsystem oder ein Druck-Schub-System, das geeignet ist, das abrasive Material in die besagte Kabine (C) zu leiten, und ein Filtersystem zum Filtern der aus der Kabine (C) ausströmenden Luft und zur Rückgewinnung des verbrauchten Materials, für das wenigstens ein geeigneter Behälter vorhanden ist.

## Revendications

1. Procédé pour le nettoyage et la désinfection d'outils chirurgicaux ou d'instruments chirurgicaux en général, comprenant l'emploi de matériel de nettoyage abrasif, émis sous pression et à vitesse élevée contre lesdits instruments pour l'abrasion et l'élimination des substances qui adhèrent aux surfaces des mêmes instruments, **caractérisé en ce que** le dit matériel de nettoyage abrasif comprend des sels de bicarbonate de sodium, des mélanges de ceux-ci.

2. Procédé selon la revendication 1, **caractérisé en ce que** ledit matériel de nettoyage est utilisé à l'état sec.

3. Procédé selon la revendication 1, **caractérisé en ce que** ledit matériel de nettoyage abrasif comprend lesdits sels de bicarbonate de sodium dissous dans l'eau au-delà de la limite de saturation de façon à former une solution comprenant des sels sous forme dissoute et/ou sous forme non dissoute, aptes à être émis sous pression et à vitesse élevée et projetés contre les instruments à nettoyer, et où ladite eau est chauffée ou non chauffée.

4. Procédé selon les revendications 1, 2 ou 3, **caractérisé en ce que** ledit matériel de nettoyage abrasif comprend aussi un ou plusieurs agents supplémentaires aux propriétés désinfectantes et/ou biocides.

5. Procédé selon la revendication 4, **caractérisé en ce que** ledit agent désinfectant et/ou biocide est présent en une concentration comprise entre 0,1 et 25 %.

6. Procédé selon les revendications précédentes, **caractérisé en ce que** ledit matériel de nettoyage abrasif comprend en outre une ou plusieurs substances supplémentaires aux propriétés désinfectantes et/ou un ou plusieurs produits médicaux à action spécifique.

7. Procédé selon les revendications précédentes, **caractérisé en ce qu'il** comprend au moins une phase finale de rinçage à l'eau des instruments et/ou une phase de soufflage pour un emploi à sec.

8. Dispositif pour le nettoyage et la désinfection d'instruments chirurgicaux selon le procédé conforme aux revendications précédentes, comprenant au moins une cabine de sablage fermée (C), où ledit matériel abrasif comprend des sels de bicarbonate de sodium, des mélanges de ceux-ci et ledit dispositif comprend en outre :
• ladite au moins une cabine de sablage fermée (C), apte à loger un ou plusieurs desdits instruments (S) à nettoyer, comprenant à son tour un boîtier avec au moins une porte d'accès pour l'introduction des instruments (S) à nettoyer ;
• au moins un système d'alimentation des sels de bicarbonate de sodium, des mélanges de ceux-ci, comprenant un réservoir contenant des sels de bicarbonate de sodium, des mélanges de ceux-ci, un conduit à travers lequel l'air pressurisé et/ou le flux d'eau s'écoule ou pousse le matériel abrasif dehors dudit réservoir pour le transporter jusqu'à une buse émettrice (U) positionnée à l'intérieur de ladite cabine (C), où ladite buse émettrice (U) éjecte ledit matériel abrasif à vitesse élevée à l'intérieur de ladite cabine de sablage (C) sur lesdits instruments à nettoyer.

9. Dispositif pour le nettoyage et la désinfection d'instruments chirurgicaux selon la revendication8, **caractérisé en ce que** ladite cabine (C) comprend au moins une ouverture avec un gant à manchette indiqué pour permettre à l'opérateur de manoeuvrer ladite buse émettrice et/ou les instruments (S) à nettoyer à l'intérieur de la cabine.

10. Dispositif pour le nettoyage et la désinfection d'instruments chirurgicaux selon les revendications 8, 9, **caractérisé en ce que** ladite buse émettrice (U) peut être déplacée d'une manière automatisée et déplacée par translation le long de trois directions spatiales et/ou tournée de façon à orienter le jet sur toute la surface des instruments (S) à nettoyer.

11. Dispositif pour le nettoyage et la désinfection d'instruments chirurgicaux selon les revendications 8, 9, **caractérisé en ce que** ladite buse émettrice (U) est fixe, en maintenant la direction du jet inchangée.

12. Dispositif pour le nettoyage et la désinfection d'instruments chirurgicaux selon les revendications 8, 9, 10 ou 11, **caractérisé en ce qu**'il comprend un ou plusieurs supports (A) pour lesdits instruments chirurgicaux (S) à l'intérieur de ladite cabine (C), aptes à bloquer les instruments (C) et les maintenir dans la position correcte durant l'opération de sablage.

13. Dispositif pour le nettoyage et la désinfection d'instruments chirurgicaux selon la revendication 12, **caractérisé en ce que** lesdits supports (A) pour les instruments (S) à nettoyer peuvent être déplacés d'une manière automatisée par rotation et/ou translation, de façon à exposer toute la surface de l'instrument (S) au jet de la buse émettrice (U).

14. Dispositif pour le nettoyage et la désinfection d'instruments chirurgicaux selon les revendications 8, 9, 10, 11, 12, 13, **caractérisé en ce qu**'il comprend : des moyens de rinçage et/ou de soufflage de l'instrument après l'opération de sablage, un système d'aspiration à vide ou un système de poussée par pression indiqué pour convoyer le matériel abrasif dans ladite cabine (C) et un système de filtration pour filtrer l'air qui sort de la cabine (C) et de récupération du matériel utilisé, dans au moins un récipient approprié.
